# EUROPEAN PATENT SPECIFICATION

(11) **EP 1 915 581 B1**
(45) Date of publication and mention of the grant of the patent: **28.09.2016**
(21) Application number: 06795634.2
(22) Date of filing: 11.08.2006
(51) Int. Cl.: F25D 17/04

(54) **A COOLING DEVICE**
KÜHLVORRICHTUNG
DISPOSITIF DE REFROIDISSEMENT

(30) Priority: 12.08.2005 TR 200503249
(43) Date of publication of application: 30.04.2008
(73) Proprietor: Arçelik Anonim Sirketi, 34950 Istanbul (TR)
(72) Inventor: GUCLU, Hande, 34950 Istanbul (TR)
(86) International application number: PCT/IB2006/052778
(87) International publication number: WO 2007/020578

(56) References cited:
- EP-A1- 0 368 839
- GB-A- 1 580 140

## Description

The present invention relates to a cooling device comprising a storage compartment containing nitrogen enriched air, purified of microorganisms for preventing the deterioration of the food items stored inside. A cooling device with a ionizer is know from GB1580140.

The most suitable medium for the growth and reproduction of microorganisms is the food stored in the open. When the necessary precautions are not taken in storing food, the microorganisms multiply rapidly leading to the deterioration and the forming of bad odors due to decaying. Storing food in cooling devices prolong the time period for the deterioration of food, however completely eradicating the microorganisms that decay food is not possible.

The shelf life of food is greatly affected by the atmosphere of the medium wherein they are kept. Particularly the ambient oxygen and the moisture content should be kept under control for storing food for a long period of time without spoilage. All the fresh vegetables and fruit take in oxygen from the medium and give off carbon dioxide and water vapor. Most of vegetables and fruits ripen after the harvest. This ripening process can be prolonged by storing the food in cold places and slowing down the metabolism and the respiration of the product. To provide the fresh food products to be stored for a longer period of time, the respiration and ripening during the storing process can be further slowed down by reducing the ambient oxygen content.

Various storing sections are designed inside the cooling compartment which are insulated from the ambient surroundings with controlled atmosphere and with enriched nitrogen gas delivered inside for the purpose of storing the food for a longer period of time. In these implementations, the nitrogen gas amount is increased while the oxygen gas amount is decreased in the medium. The reproduction of aerobic microorganisms is prevented since the amount of oxygen gas is decreased and the deterioration of the food is slowed down. Furthermore, since the nitrogen gas does not react under normal conditions, there is not an adverse effect on the stored food items.

The enriched nitrogen is produced by the nitrogen generator in the cooling devices that comprise compartments insulated from the outer environment. The nitrogen generator is operated by a control card in alternating on/off positions so as to keep the nitrogen gas in the storing compartment within certain values. When it is in the "on" position, the oxygen gas and humidity levels are reduced, in the "off" position oxygen gas and humidity levels are increased in the storing compartment. When the nitrogen generator is off, the increase of oxygen gas and humidity levels creates a favorable medium for the growth of especially the aerobic microorganisms in the storing compartment.

The object of the present invention is to design a cooling device comprising an ionizer that enables to eradicate the microorganisms in the storage compartment containing enriched nitrogen.

The cooling device designed to fulfill the objectives of the present invention, explicated in the first and the respective claims, comprises a storage compartment, insulated from the ambient surroundings, containing nitrogen-enriched air, wherein the air inside the compartment is monitored by controlling the humidity and gas content, and an ionizer, arranged within this storage compartment, providing to remove the mi-croorganisms within the storage compartment and preventing the formation of a favorable medium for the reproduction of the microorganisms, operated by a control card when the oxygen and humidity levels within the storage compartment tend to increase.

In another embodiment of the present invention, the ionizer is operated when the nitrogen generator is off, preventing the formation of a medium for the reproduction of microorganisms resulting from the non-operation of the nitrogen generator leading to the deterioration of the stored foods. Accordingly when the nitrogen generator is off, the reproduction of the aerobic microorganisms is restricted providing the storing of food for a longer period of time in the storage compartment.

The operation of the ionizer is controlled by a control card which evaluates the data received from various sensors. In embodiments wherein sensors are not present, the control card controls the operation of the ionizer by using the timing information.

The cooling device designed to fulfill the objectives of the present invention is illustrated in the attached figures, where:

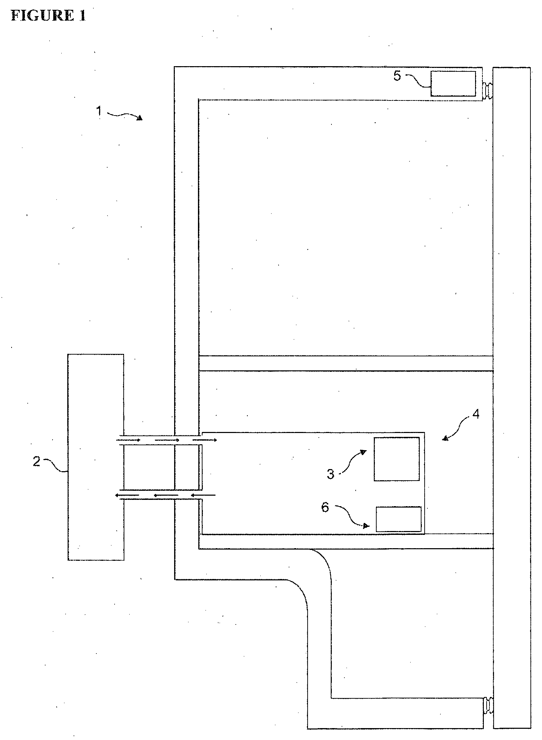

Figure 1 - is the schematic view of a cooling device.

The elements shown in figures are numbered as follows:
1. Cooling device
2. Nitrogen generator
3. Ionizer
4. Storage compartment
5. Control card
6. Sensor

The cooling device (1) of the present invention comprises a nitrogen generator (2) which generates nitrogen (N2) enriched air, a storage compartment (4) into which the nitrogen-enriched air generated by the nitrogen generator (2) is delivered, an ionizer (3), disposed within the storage compartment (4), which ionizes the air in its vicinity within the storage compartment (4), providing to remove the microorganisms within the storage compartment (4) and preventing the formation of a favorable medium for the reproduction of the microorganisms and a control card (5) that operates the ionizer (3) (see Figure 1).

The cooling device (1) comprises one or more sensors (6) that detect the ratio of the oxygen gas and the ratio of humidity within the storage compartment (4).

The nitrogen generator (2) comprises a pump (not shown in the figures) that provides the suction and pumping of the air. The air sucked in by the pump is delivered to the nitrogen generator (2). The nitrogen in the air delivered to the nitrogen generator (2) is separated from air by various methods and nitrogen-enriched air is obtained. The nitrogen-enriched air is delivered to the storage compartment (4) by the pump and the rest is discharged from the nitrogen generator (2).

The ionizer (3) comprises a high voltage source fed by the network power supply that increases the voltage to preferably 5.000 - 10.000 DC V and an electrode that is connected to this high voltage source. When high voltage is applied to the electrode, the air molecules in the vicinity of the electrode are ionized and the negative ion amount inside the medium is increased.

The nitrogen is decomposed from the aspirated air by the operation of the nitrogen generator (2) and the decomposed nitrogen gas is delivered to the insulated storage compartment (4). The nitrogen generator (2) is operated by a control card (5) at certain intervals predetermined by the manufacturer for keeping the nitrogen gas ratio in the storage compartment (4) within certain limits. The oxygen and humidity levels inside the storage compartment (4) decrease when the nitrogen generator (2) is on, and increase when it is off.

The ionizer (3) is started by the control card (5) when the oxygen and humidity levels show an upward trend detected by the sensors (6), providing to remove the microorganisms from the storage compartment (4) and also taking under control the ratio of the oxygen gas and humidity levels that help in the reproduction of the microorganisms.

When the ionizer (3) is operated, an electric field is created between the electrode and the surrounding air as a result of the high voltage, the molecules in the air are separated into ions and the negative ion amount around the electrode is increased. The negative ions provide the positively charged microorganisms in the storage compartment (4) to become heavier and sink. During the ionization process of the air molecules, the oxygen gas in the air is also ionized. Thus, the amount of the oxygen gas that is effective in the growth of the microorganisms in the storage compartment (4) is also decreased.

In another embodiment of the present invention, the sensors (6) are not used and the ionizer (3) is started by the control card (5) when the nitrogen generator (2) is off. In this embodiment, the ionizer (3) is turned off when the nitrogen generator (2) starts to operate.

In another embodiment of the present invention, where the sensors (6) are not used, the ionizer (3) is operated by the control card (5) at time intervals and for time periods predetermined by the manufacturer.

In another alternative embodiment of the present invention, preferably one wall of the storage compartment (4) is made of metal. The microorganisms surrounded by the negatively charged ions in the environment are collected on this wall due to the electrical charge difference and are discharged together with the water condensed on this wall out of the storage compartment (4).

By way of the present invention, particularly when the nitrogen generator (2) is off, by operating the ionizer (3) by the control card (5) in cases when the oxygen gas amount and the humidity increase favoring the growth of the microorganisms, the eradication of the microorganisms and formation of an unfavorable medium for their reproduction are provided.

## Claims

1. A cooling device (1) comprising: a nitrogen generator (2) generating nitrogen (N2) enriched air, a storage compartment (4) into which the nitrogen-enriched air is delivered by the nitrogen generator (2), and an ionizer (3) disposed inside the storage compartment (4) increasing the amount of negatively charged ions by ionizing the air in its vicinity and providing the formation of an unfavorable medium for the reproduction of the microorganisms thereby removing the microorganisms from the storage compartment (4).

2. A cooling device (1) as in Claim 1, comprising one or more sensors (6) that detect the ratio of the oxygen gas and humidity in the storage compartment (4), a control card (5) that controls the nitrogen generator (2) and the ionizer (3), and an ionizer (3) that is started by the control card (5) when the oxygen and humidity levels show an upward trend.

3. A cooling device (1) as in Claim 1, comprising an ionizer (3) that is operated by a control card (5) when the nitrogen generator (2) is off.

4. A cooling device (1) as in Claim 1, comprising an ionizer (3) that is operated by a control card (5) when the nitrogen generator (2) is off for time periods at certain time intervals pre-determined by the manufacturer.

## Patentansprüche

1. Kühlvorrichtung (1), umfassend einen Stickstoffgenerator (2), der mit Stickstoff (N₂) angereicherte Luft erzeugt, ein Aufbewahrungsfach (4), in das die mit Stickstoff angereicherte Luft von dem Stickstoffgenerator (2) geleitet wird, und einen Ionisierer (3), der im Inneren des Aufbewahrungsfachs (4) angeordnet ist und die Mengen an negativ geladenen Ionen erhöht, indem er die Luft in seiner Umgebung ionisiert, und für die Bildung eines ungünstigen Mediums für die Vermehrung der Mikroorganismen schafft, so dass die Mikroorganismen aus dem Aufbewahrungsfach (4) beseitigt werden.

2. Kühlvorrichtung (1) nach Anspruch 1, umfassend einen oder mehrere Sensoren (6), die das Verhältnis von Sauerstoffgas und Feuchtigkeit im Aufbewahrungsfach (4) erkennen, eine Steuerkarte (5), die den Stickstoffgenerator (2) und den Ionisierer (3) steuert, und einen Ionisierer (3), der von der Steuerkarte (5) gestartet wird, wenn die Sauerstoff- und Feuchtigkeitspegel einen Aufwärtstrend zeigen.

3. Kühlvorrichtung (1) nach Anspruch 1, umfassend einen Ionisierer (3), der von einer Steuerkarte (5) betrieben wird, wenn der Stickstoffgenerator (2) ausgeschaltet ist.

4. Kühlvorrichtung (1) nach Anspruch 1, umfassend einen Ionisierer (3), der von einer Steuerkarte (5) betrieben wird, wenn der Stickstoffgenerator (2) für Zeitspannen in bestimmten Zeitintervallen, die vom Hersteller vorgegeben werden, ausgeschaltet ist.

## Revendications

1. Un dispositif de refroidissement (1) comprenant un générateur d'azote (2) qui génère de l'air enrichi en azote (N₂), un compartiment de stockage (4) dans lequel l'air enrichi en azote est délivré par le générateur d'azote (2), et un ioniseur (3) qui est disposé dans le compartiment de stockage (4), qui augmente la quantité d'ions chargés négativement en ionisant l'air dans son voisinage et qui permet la formation d'un milieu défavorable à la reproduction des micro-organismes, donc éliminant les micro-organismes dans le compartiment de stockage (4).

2. Un dispositif de refroidissement (1) selon la Revendication 1, comprenant un ou plusieurs capteurs (6) qui détectent le rapport de l'oxygène et l'humidité dans le compartiment de stockage (4), une carte de commande (5) qui commande le générateur d'azote (2) et l'ioniseur (3), et un ioniseur (3) qui est démarré par la carte de commande (5) lorsque les niveaux d'oxygène et d'humidité montrent une tendance à la hausse.

3. Un dispositif de refroidissement (1) selon la Revendication 1, comprenant un ioniseur (3) qui est fonctionné par une carte de commande (5) lorsque le générateur d'azote (2) est éteint.

4. Un dispositif de refroidissement (1) selon la Revendication 1, comprenant un ioniseur (3) qui est fonctionné par une carte de commande (5) lorsque le générateur d'azote (2) est éteint pendant des périodes de temps à des certains intervalles de temps prédéterminés par le fabricant.
